# FASCICULE DE BREVET EUROPEEN

(11) **EP 3 113 850 B1**
(45) Date de publication et mention de la délivrance du brevet: **26.06.2019**
(21) Numéro de dépôt: 15709299.0
(22) Date de dépôt: 20.02.2015
(51) Int. Cl.: A61Q 19/02, A61K 8/97, A61K 8/36, A61K 8/368

(54) **COMPOSITION COSMETIQUE ECLAIRCISSANTE**
HAUTAUFHELLENDE KOSMETIKZUSAMMENSETZUNG
SKIN-BRIGHTENING COSMETIC COMPOSITION

(30) Priorité: 06.03.2014 FR 1451817
(43) Date de publication de la demande: 11.01.2017
(73) Titulaire: Laboratoires M & L, 04100 Manosque En Provence (FR)
(72) Inventeur: PORTES, Pascal, F-13540 Puyricard (FR); LEMAIRE, Géraldine, F-04210 Valensole (FR); TOUREL, Cécile, F-13090 Aix En Provence (FR)
(74) Mandataire: Novagraaf Technologies
(86) Numéro de dépôt international: PCT/FR2015/050428
(87) Numéro de publication internationale: WO 2015/132501

(56) Documents cités:
- FR-A1- 2 784 294
- FR-A1- 2 876 284
- FR-A1- 2 939 669
- US-A1- 2008 008 673
- US-A1- 2008 159 970
- US-A1- 2012 177 586

## Description

### Domaine technique

La présente invention se rapporte également à l'utilisation cosmétique d'une composition cosmétique ou dermatologique comprenant au moins un extrait de reine des prés, au moins un extrait de mûrier blanc et de l'acide salicylique.

La présente invention se rapporte également à un procédé cosmétique non thérapeutique pour traiter les taches de vieillesse, les mélasmas, les lentigos et/ou le masque de grossesse ou chloasma.

La présente invention trouve une application notamment dans les domaines cosmétique et dermatologique.

Dans la description ci-dessous, les références entre crochets ([ ]) renvoient à la liste des références présentées à la fin du texte.

### Etat de la technique

La peau est un organe à part entière qui joue un rôle essentiel, non seulement dans la protection du corps contre les agressions extérieures, mais aussi sur le plan esthétique et émotionnel. La peau est un organe d'échange. Son aspect visuel mais aussi tactile occupe une place très importante.

La peau comprend différents compartiments ainsi que différents types cellulaires. En particulier, les mélanocytes présents dans la couche basale de l'épiderme sont connus pour produire notamment un pigment : la mélanine de couleur sombre. Cette mélanine s'exporte via de petites vésicules appelées mélanosomes vers les kératinocytes voisins provoquant ainsi une coloration brune de la peau. La peau est soumise au cours du temps à des agressions extérieures, tels que les rayonnements Ultra-Violet, pollution etc. susceptibles de modifier son aspect. En particulier, il est bien connu que, les rayonnements du soleil et en particulier les rayonnements Ultra- Violet induisent une synthèse de mélanine provoquant un « assombrissement » ou coloration de la peau. Depuis plusieurs décennies dans les régions occidentales, le besoin d'un aspect « bonne mine » correspondant notamment à un teint hâlé et/ou à une peau bronzée est un signe de bonne santé et/ou de séduction tant pour les hommes que pour les femmes. Toutefois, cette volonté a entrainé un besoin de bronzer en toute saison, par exemple via des séances UV en institut, susceptible de provoquer un vieillissement de la peau et/ou la formation de taches cutanées dues à une pigmentation accrue de la peau. En d'autres termes, une pigmentation irrégulière peut survenir lors ou après exposition au soleil formant, notamment des irrégularités de la pigmentation de la peau et un aspect inesthétique.

Des problèmes d'hyperpigmentation de la peau peuvent également apparaitre dans des cas de photosensibilisation et/ou de cicatrisation post lésionnelle provoquant également un aspect inesthétique.

Par ailleurs, chez les femmes lors de la grossesse, une modification hormonale, notamment une augmentation des taux d'hormones féminines, entraîne en même temps une forte augmentation de la synthèse de la mélanine par les mélanocytes. En d'autres termes l'hyperpigmentation de la grossesse ou chloasma est notamment liée à une hyperactivité des mélanocytes due à la forte production d'hormones sexuelles. Durant cette période, si la peau est exposée au soleil, il peut y avoir une hyperpigmentation de celle-ci, et apparition de taches, notamment au niveau du visage désigné communément comme « masque de grossesse ». Ces taches de pigmentation peuvent perdurer dans le temps et ceci même après l'accouchement et un retour à des taux normaux d'hormones féminines. Ces taches peuvent également se réduire dans le temps mais réapparaitront spontanément à chaque exposition au soleil / UV faisant ainsi réapparaitre le masque de grossesse et donc une disparité concernant la pigmentation de la peau du visage.

Des taches pigmentaires peuvent apparaitre chez les sujets âgés, par exemple les lentigos séniles ou taches de vieillesse provoquant un aspect tacheté de la peau. Ces différences de pigmentation sont notamment dues à une concentration localisée de mélanine à la surface de la peau.

Il existe donc un réel besoin de trouver une composition ou un produit permettant notamment de traiter les défauts de pigmentation de la peau et ceci quelque soit leur origine, à savoir notamment une augmentation de la production de mélanine due à une modification hormonale, une surexposition aux U.V. et/ou au soleil, un vieillissement de la peau.

Par ailleurs, dans les régions asiatiques, notamment en Chine, Japon et Corée du Sud, l'aspect de la peau peut être encore considéré comme un marqueur socio-économique. En effet, dans ces régions, une peau brunie par le soleil peut être considérée comme un élément d'appartenance à une classe sociale considérée comme inférieure, par exemple personne travaillant sur des chantiers/à l'extérieur tel que des agriculteurs etc. En d'autres termes, les conditions de vie déterminent le temps d'exposition au soleil et serait un reflet du niveau socio-économique. En outre, depuis des siècles dans les régions asiatiques, l'aspect peau blanche correspond à un signe de beauté, par exemple pour les geishas au Japon, et/ou d'appartenance à une classe sociale aisée. Enfin, il faut rappeler que l'apparition des taches pigmentaires est un des signes cliniques majeurs du vieillissement des peaux asiatiques, au même titre que les rides et ridules pour les populations occidentales.

Aussi, il existe un besoin, de trouver une composition ou un produit permettant notamment d'éclaircir la peau, par exemple en diminuant la production de mélanine ou en favorisant sa dégradation.

Il existe dans l'état de la technique des compositions dites éclaircissantes qui ont pour but de diminuer la coloration de la peau, par exemple via l'inhibition d'enzymes responsables de la synthèse de la mélanine.

Par exemple parmi les compositions connues pour freiner ou inhiber la production de mélanine par les mélanocytes, on peut citer les compositions comprenant de l'hydroquinone et de nombreux produits dont la structure moléculaire est proche de celle de la tyrosine.

Toutefois, ces compositions présentent de nombreux effets secondaires, tels qu'un pouvoir irritant de la peau et/ou allergisant accru. En outre, ces compositions ont des efficacités relatives, en particulier, concernant le traitement des masques de grossesse et/ou des taches cutanées dues, notamment au soleil et/ou au vieillissement. Ces efficacités relatives peuvent elles-mêmes être à l'origine d'un aspect inesthétique allant donc à l'encontre de l'objectif premier.

Il existe donc un réel besoin de trouver une nouvelle composition cosmétique palliant ces défauts, inconvénients et obstacles de l'art antérieur, en particulier une composition cosmétique permettant de réduire les taches d'hyperpigmentation, les masques de grossesses etc. tout en réduisant les effets secondaires connus des compositions de l'art antérieur tels que l'irritation cutanée et/ou le pouvoir allergisant liés aux compositions éclaircissantes connues.

### Description de l'invention

La présente invention a précisément pour but de répondre à ces besoins en fournissant une composition cosmétique comprenant au moins un extrait de reine des prés, au moins un extrait de mûrier blanc et de l'acide salicylique.

La présente invention se rapporte également à l'utilisation cosmétique et/ou dermatologique de cette composition. En particulier, la présente invention se rapporte à l'utilisation cosmétique et/ou dermatologique de la composition cosmétique selon l'invention pour éclaircir la peau, traiter les taches de vieillesse, les mélasmas, les lentigos et/ou le masque de grossesse ou chloasma.

Les inventeurs de la présente invention sont en effet les tous premiers à avoir mis en évidence, de manière tout à fait inattendue que la composition cosmétique comprenant au moins un extrait de reine des prés, au moins un extrait de mûrier blanc et de l'acide salicylique permet avantageusement et de manière synergique de diminuer la pigmentation de la peau.

En outre, les inventeurs de la présente invention sont en effet les tous premiers à avoir mis en évidence, de manière tout à fait inattendue que la composition cosmétique comprenant un extrait de reine des prés, au moins un extrait de mûrier blanc et de l'acide salicylique permet avantageusement et de manière synergique d'inhiber la production de mélanine.

De plus, les inventeurs de la présente invention, de part l'effet synergique des composants présents dans la composition selon l'invention, ont montré que la composition permet avantageusement d'éclaircir la peau tout en présentant une très bonne tolérance cutanée.

Dans la présente par « extrait » on entend un extrait obtenu par exemple à partir d'une plante et/ou d'une partie d'une plante. Il peut s'agir par exemple d'un extrait obtenu à partir de fleurs ou sommités fleuries, feuilles, graines, racines, tiges, péricarpe. Il peut s'agir par exemple d'un extrait obtenu à partir de fleurs entières.

Dans la présente par « reine des prés » on entend une espèce de plantes vivaces de la famille des *Rosaceae* du genre *Filipendula.* En particulier, il peut s'agir de *Filipendula ulmaria,* de *Spiraea ulmaria.* De préférence dans la présente la reine des prés peut être *Spiraea ulmaria.*

Selon l'invention, l'extrait de reine des prés peut être un extrait obtenu à partir de fleurs ou sommités fleuries, feuilles, graines, racines, tiges, péricarpe. Avantageusement, il peut s'agir par exemple d'un extrait de reine des prés obtenu à partir de fleurs entières. Selon l'invention, l'extrait de reine des prés peut être un extrait choisi parmi un extrait aqueux, un extrait huileux, un extrait glycolique, une huile essentielle ou un mélange de ceux-ci.

Dans la présente par « extrait aqueux » on entend tout extrait aqueux obtenu par tout procédé connu de l'homme du métier. Par exemple, il peut être obtenu par extraction aqueuse, par hydrodistillation, extraction hydro-glycérinée ou hydro-alcoolique ou hydro-glycolique.

Selon l'invention, l'extrait aqueux de reine des près peut être tout extrait aqueux de reine des près connu de l'homme du métier. Il peut s'agir par exemple d'un extrait disponible dans le commerce, par exemple un extrait d'inflorescences de reine des prés commercialisé par la société *Ichimaru Pharcos* sous la référence commerciale *Meadowsweet Liquid BG* (nom INCI: *Spiraea Ulmaria* Flower Extract (and) water (and) butylene glycol), l'extrait commercialisé par la société Vege Tech sous la référence commerciale VT-053 Extract of Meadowsweet.

Dans la présente par « extrait huileux » on entend un extrait riche en acide gras et/ou en matière grasse et/ou un extrait non hydrosoluble.

Selon l'invention, l'extrait huileux peut être tout extrait huileux obtenu par tout procédé connu de l'homme du métier. Par exemple, il peut être obtenu par extraction huileuse par micro-ondes, par hyperfréquence, par extraction supercritique, par macération dans un solvant huileux, par extraction huileuse assistée par ultrasons.

Selon l'invention, l'extrait huileux de reine des près peut être tout extrait huileux de reine des prés connu de l'homme du métier. Il peut s'agir par exemple d'un extrait huileux disponible dans le commerce, par exemple un extrait commercialisé par la société Vevy Europe sous la référence commerciale Lipoplastidine Spirea, un extrait commercialisé par la société Active Organics Europe sous les références commerciales Actiphyte of Meadowsweet Lipo S, CO Actiphyte of Meadowsweet Lipo O ou CO Actiphyte of Meadowsweet Lipo Sun. Il peut s'agir par exemple d'un extrait huileux biologique de reine des prés obtenu par extraction micro-ondes assistée par ultrasons selon un procédé comprenant, par exemple une première étape de broyage de fleurs entières de reine des prés suivie d'une extraction assistée par ultrasons et par micro-ondes, puis une clarification et enfin une étape de filtration afin de récupérer l'extrait huileux.

Dans la présente par « huile essentielle » on entend toute huile essentielle obtenue par tout procédé connu de l'homme du métier. Par exemple, il peut s'agir d'une huile essentielle obtenue par hydrodistillation.

Selon l'invention, l'huile essentielle de reine des prés peut être toute huile essentielle de reine des prés connue de l'homme du métier. Il peut s'agir par exemple d'une huile essentielle disponible dans le commerce, par exemple une huile essentielle commercialisée par la société *Stever* et/ou de la boutique en ligne IBOA

Selon l'invention, l'extrait de reine des prés peut être présent dans la composition par exemple à une concentration de 0,01 à 10% en poids par rapport au poids total de ladite composition, par exemple de 0,01 à 5%, de 0,5 à 1 % en poids par rapport au poids total de ladite composition.

Dans la présente par « mûrier blanc » on entend une espèce de plantes de la famille des *Moraceae* du genre *Morus.* En particulier, il peut s'agir par exemple de *Morus Alba.*

Selon l'invention, l'extrait de mûrier blanc peut être un extrait obtenu à partir de fleurs ou sommités fleuries, feuilles, graines, racines, tiges, péricarpe. Avantageusement, il peut s'agir par exemple d'un extrait de mûrier blanc obtenu à partir de racines.

Selon l'invention l'extrait de mûrier blanc peut être un extrait choisi parmi un extrait aqueux, un extrait huileux, un extrait glycolique, ou un mélange de ceux-ci.

Selon l'invention, l'extrait aqueux de mûrier blanc peut être tout extrait aqueux de mûrier blanc connu de l'homme du métier. Il peut s'agir par exemple d'un extrait disponible dans le commerce, par exemple un extrait commercialisé par la société *Ichimaru Pharcos* sous la référence commerciale « *Souhakuhi Liquid BG* » (nom INCI *:Morus alba* root extract (and) water (and) butylene glycol), d'un extrait commercialisé par la société Medical Nanomaterial Institute sous la référence commerciale « Morus Alba Leaf Supercritical Extract », d'un extrait commercialisé par la société Draco natural Products sous la référence commerciale « Mulberry Extract ».

Selon l'invention, l'extrait huileux de mûrier blanc peut être tout extrait huileux de mûrier blanc connu de l'homme du métier. Il peut s'agir par exemple d'un extrait huileux disponible dans le commerce, par exemple un extrait commercialisé par la société Active Organics sous la référence catalogue « Actiphyte of Mulberry Bark Lipo S », par la société Maruzen Pharmaceuticals sous la référence catalogue « Mulberry Extract-JO ».

Selon l'invention, l'extrait glycolique de mûrier blanc peut être tout extrait glycolique de mûrier blanc connu de l'homme du métier. Il peut s'agir par exemple d'un extrait disponible dans le commerce, par exemple un extrait commercialisé par la société Active Organics sous la référence commerciale « Actiphyte of Mulberry Bark BG50P » et/ou « Actiphyte of Mulberry leaf BG50P », par la société Natural Solution sous la référence commerciale Odi Extract, par la société Bioland sous la référence commerciale « Morus alba twig extract » et/ou par la société E.U.K, sous la référence commerciale « Far East Extract Tohasaku ».

Selon l'invention, l'extrait de mûrier blanc peut être présent dans la composition cosmétique à une concentration de 0,01 à 10 %, par exemple de 0,01 à 5%, de 0,5 à 1% en poids par rapport au poids total de ladite composition.

Dans la présente par « acide salicylique » on entend l'acide 2-hydroxybenzoïque de formule (I) suivante :

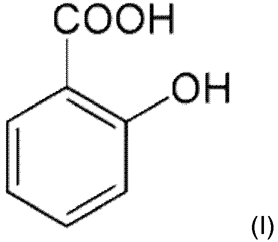

Selon l'invention, l'acide salicylique peut être présent dans la composition cosmétique à une concentration de 0,001 à 2 %, par exemple de 0,003 à 0,1%, de 0,0045 à 0,1% en poids par rapport au poids total de ladite composition.

Avantageusement, les inventeurs de la présente invention ont démontré de manière surprenante qu'une composition comprenant à la fois un extrait de mûrier blanc, un extrait de reine des prés et de l'acide salicylique permet de diminuer les taches pigmentaires tel que les taches de vieillesse, les masques de grossesse, les mélasmas et les lentigos. En outre, les inventeurs ont démontré de manière surprenante que la composition selon l'invention diminue de manière synergique la synthèse de la mélanine permettant ainsi de prévenir, par exemple la réapparition de masque de grossesse, ou de traiter tout désordre pigmentaire, par exemple les taches pigmentaires, le mélasma et/ou tout désordre pigmentaire connu de l'homme du métier.

Selon l'invention la composition peut comprendre en outre de la Vitamine C également désigné acide ascorbique ou ses dérivés.

Selon l'invention, les dérivés de l'acide ascorbique peuvent être choisi par exemple dans le groupe comprenant l'acide ascorbique 2-glucosidique, ou glucoside d'ascorbyle et l'acide érythorbique

Selon l'invention, l'acide ascorbique et/ou au moins un de ses dérivés peut être présent dans la composition cosmétique à une concentration de 0,01 à 10 %, par exemple de 0,1 à 2% en poids par rapport au poids total de ladite composition.

Avantageusement, les inventeurs ont démontré que la composition cosmétique comprenant en outre de l'acide ascorbique et/ou au moins un de ses dérivés permet de renforcer l'effet éclaircissant de la composition en augmentant l'inhibition de la synthèse de mélanine.

Avantageusement, les inventeurs ont démontré que la composition cosmétique comprenant un extrait de mûrier blanc, un extrait de reine des prés, de l'acide salicylique et en outre de l'acide ascorbique et/ou au moins un de ses dérivés permet d'obtenir un effet éclaircissant via notamment l'inhibition de la synthèse de mélanine.

En d'autres termes, la composition selon l'invention permet avantageusement d'obtenir une composition présentant une efficacité éclaircissante adaptée tout en évitant/supprimant l'apparition d'effets secondaires, notamment d'intolérance cutanée.

Selon l'invention, la composition cosmétique de la présente invention peut comprendre en outre au moins un agent actif additionnel choisi, par exemple parmi un agent éclaircissant complémentaire, un agent anti-âge, un agent hydratant, un agent apaisant, un agent agissant sur la micro-circulation, un ou plusieurs agents antioxydants, un agent raffermissant, un agent tenseur ou un mélange de ces agents. Ces agents peuvent être ceux couramment utilisés dans les produits cosmétiques et/ou dermatologiques. Un exemple d'agent anti-âge utilisable peut être par exemple le silicium. Des exemples d'agents hydratants utilisables peuvent être par exemple la glycérine, l'acide hyaluronique ou le D-Panthenol. Des exemples d'agents apaisants utilisables peuvent être par exemple l'alpha bisabolol ou l'allantoïne. Des exemples d'agents agissant sur la micro-circulation peuvent être par exemple l'escine ou l'arginine. Des exemples d'agents raffermissants peuvent être par exemple un dérivé de silicium, des agents tenseurs par exemple des protéines d'amande. Ce ou ces agent(s) peuvent être utilisé(s) dans la composition de la présente invention par exemple aux concentrations usuellement utilisées et connues de l'homme du métier dans les compositions cosmétiques ou dermatologiques. La composition cosmétique de la présente invention peut également comprendre en outre au moins un agent actif additionnel choisi, par exemple parmi un agent conditionneur, un agent réparateur. Ces agents peuvent être ceux couramment utilisés dans les produits cosmétiques et/ou dermatologiques. Des exemples d'agents conditionneurs utilisables peuvent être par exemple les protéines de maïs, de riz, les quaterniums, des protéines hydrolysées quaternisées. Des agents réparateurs peuvent être par exemple des céramides, des huiles végétales d'amande, d'argan, du beurre de karité.

La composition cosmétique selon l'invention peut comprendre un ou plusieurs supports cosmétiquement acceptable(s). Dans la présente, par « support cosmétiquement acceptable» on entend tout support cosmétique connu de l'homme du métier, il peut s'agir par exemple de tout support cosmétique pouvant être cité dans le dictionnaire INCI (International Nomenclature of Cosmetic Ingrédients) publié par le PCPC (Personal Care Products Council).

La composition cosmétique selon l'invention peut comprendre un ou plusieurs adjuvants connus de l'homme du métier. Il peut s'agir par exemple d'un ou plusieurs adjuvant(s) choisi(s) parmi des agents de type esters, des agents hydratants, des agents émollients, des agents épaississants minéraux, des agents épaississants organiques, associatifs ou non, des filtres solaires organiques hydrosolubles et liposolubles, des filtres solaires minéraux, des composés siliconés, des parfums, des conservateurs, des céramides, et pseudo-céramides, des vitamines et les provitamines, des protéines, des agents séquestrants, des agents alcanisants, des agents acidifiants, des agents réducteurs, des agents oxydants, des charges minérales, des colorants ou tout autre adjuvant pouvant être cité dans le dictionnaire INCI (International Nomenclature of Cosmetic Ingrédients) publié par le PCPC (Personal Care Products Council).

Selon l'invention, la composition cosmétique peut, par exemple, se présenter sous toute forme connue de l'homme du métier. Il peut s'agir, par exemple d'une émulsion huile-dans-l'eau, eau-dans-l'huile, eau dans silicone, d'une émulsion multiple, d'une microémulsion, d'une nano-émulsion, d'une émulsion solide, d'un gel aqueux ou hydro-alcoolique, d'une crème, d'un gel, d'un lait, d'une lotion, d'une pommade, d'une huile, d'un baume, d'un onguent, d'un masque, d'une poudre, d'un support imbibé, par exemple un patch transdermique, d'une lotion aqueuse ou hydroalcoolique et/ou une cire, d'un produit de maquillage, par exemple un fond de teint, d'un shampooing, d'un après-shampooing, d'un masque, sérum, lotion capillaire. De préférence, la composition cosmétique selon l'invention peut être sous une forme choisie parmi une crème, un gel, une lotion, un onguent, une huile, un lait, un masque, un sérum, un patch.

La composition cosmétique ou dermatologique de l'invention peut être obtenue par tout procédé approprié connu de l'homme du métier pour la fabrication d'une composition cosmétique et/ou dermatologique. Il peut s'agir, par exemple d'un simple mélange. Il peut s'agir d'un mélange de matières tensioactives dans de l'eau. Il peut s'agir également, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), procédé classiquement utilisé par l'homme de l'art pour obtenir des émulsions huile dans eau dont les gouttelettes dispersées sont particulièrement fines, par exemple avec un diamètre de 0,1 à 1 µm.

Selon l'invention, la composition cosmétique peut être une composition utilisée dans un soin cosmétique, par exemple un soin cosmétique ou dans un traitement cosmétique non thérapeutique pour éclaircir la peau et/ou traiter les taches de vieillesse et/ou traiter les mélasmas et/ou traiter les lentigos et/ou traiter le masque de grossesse ou chloasma.

Dans la présente par « soin cosmétique » on entend par exemple l'application cutanée et/ou sur les cheveux, poils ou cils d'une composition selon l'invention.

La présente invention a également pour objet un procédé de traitement cosmétique non thérapeutique comprenant l'application sur la peau d'une composition cosmétique selon l'invention. La présente invention a également pour objet un procédé de traitement cosmétique comprenant l'application sur la peau d'une composition cosmétique comprenant au moins un extrait de reine des prés, au moins un extrait de mûrier blanc et de l'acide salicylique. Avantageusement, le procédé de traitement cosmétique comprend l'application d'une composition cosmétique comprenant au moins un extrait de reine des prés, au moins un extrait de mûrier blanc, de l'acide salicylique et de la vitamine C ou ses dérivés.

L'extrait de mûrier blanc, l'extrait de reine des prés, l'acide salicylique, la vitamine C ou ses dérivés sont tels que définis ci-dessus.

La composition cosmétique peut être celle définie précédemment.

L'application peut être toute application souhaitée par l'utilisateur/l'utilisatrice, par exemple une application quotidienne, biquotidienne, hebdomadaire et/ou bi-mensuelle. Il peut s'agir par exemple d'une application une fois par jour, deux fois par jour ou plus.

La présente invention a également pour objet une trousse de traitement cosmétique non-thérapeutique de la peau comprenant une composition cosmétique selon l'invention et un support comportant des instructions pour l'emploi de la composition cosmétique sur la peau.

Le support comportant des instructions pour l'emploi de la composition cosmétique selon l'invention peut être un manuel ou une notice qui peut avoir notamment pour fonction d'expliquer à l'utilisateur la manière et la fréquence d'application sur la peau de la composition de ladite présente invention.

Selon l'invention, la trousse de soin cosmétique peut également comprendre un applicateur, par exemple une spatule, une brosse, un applicateur dynamique, par exemple un dispositif de massage imprégné de la composition cosmétique de l'invention.

D'autres avantages pourront encore apparaître à l'homme du métier à la lecture des exemples ci-dessous, illustrés par les figures annexées, donnés à titre illustratif.

### Brève description des figures

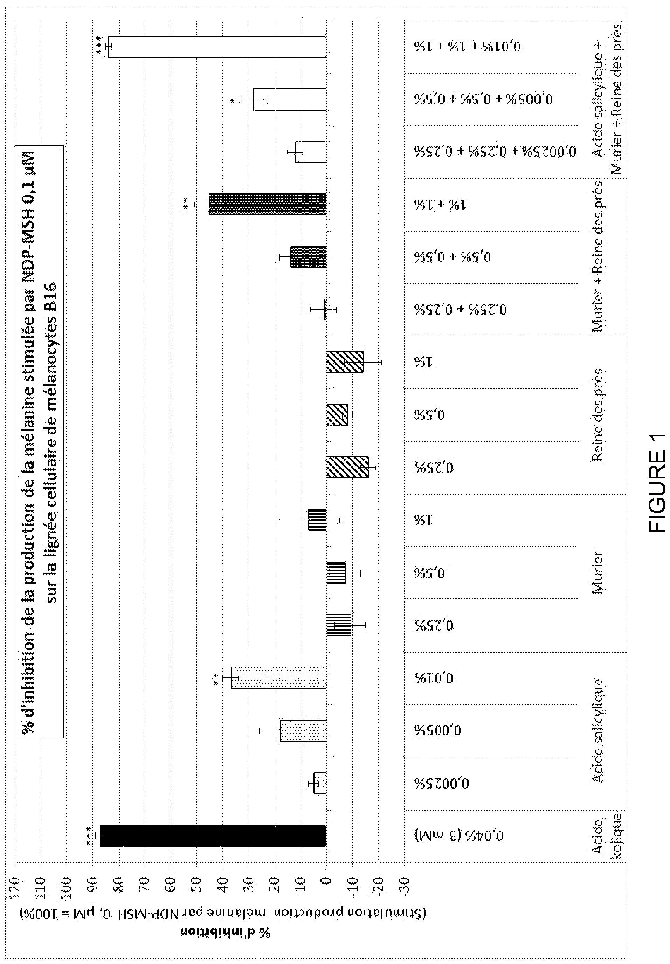

- La figure 1 représente un diagramme en bâton représentant le pourcentage d'inhibition de la production de mélanine en présence d'acide kojique, d'acide salicylique, d'extrait de mûrier blanc, d'extrait de reine des prés, d'un mélange d'extraits de mûrier blanc et de reine des prés, d'un mélange d'extraits de mûrier blanc, d'extrait de reine des prés et d'acide salicylique.

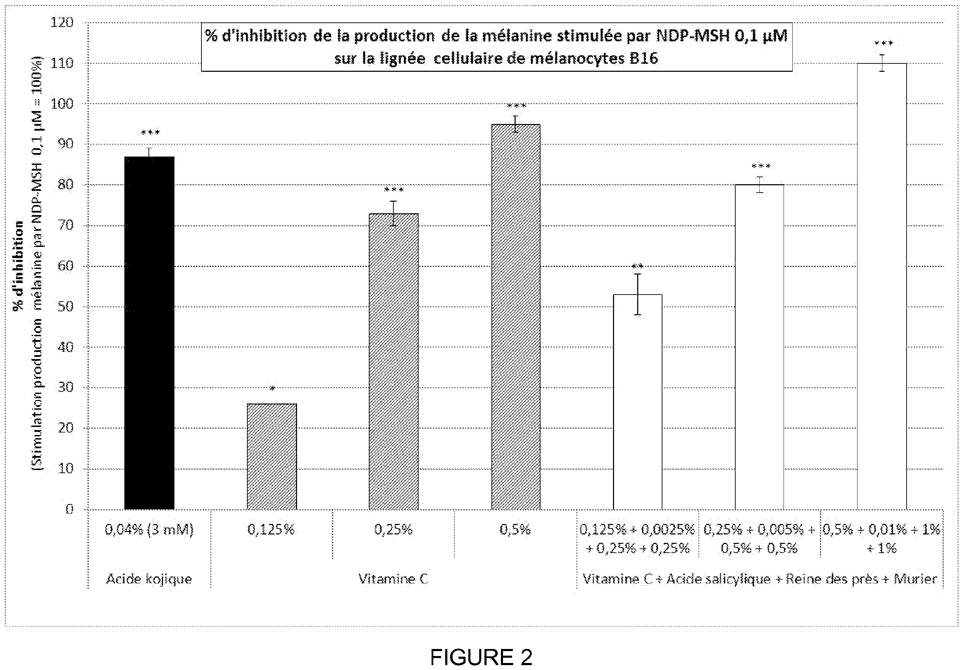

- La figure 2 représente un diagramme en bâton représentant le pourcentage d'inhibition de la production de mélanine en présence d'acide kojique, de vitamine C, et d'un mélange d'extraits de mûrier blanc, d'extrait de reine des prés, d'acide salicylique et de vitamine C.

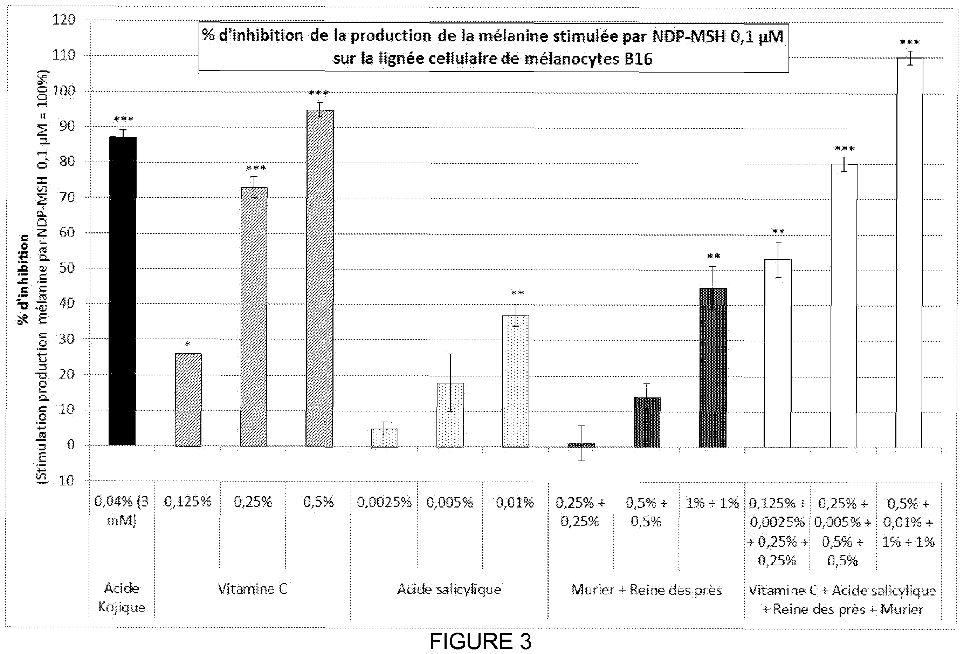

- La figure 3 représente un diagramme en bâton représentant le pourcentage d'inhibition de la production de mélanine en présence d'acide kojique, de vitamine C, d'acide salicylique, d'un mélange d'extraits de mûrier blanc et de reine des prés, et d'un mélange d'extrait de mûrier blanc, d'extrait de reine des prés, d'acide salicylique et de vitamine C.

### EXEMPLES

### Exemple 1 : Composition cosmétique éclaircissante

Les compositions ci-dessous ont été préparées selon le procédé suivant :
- La gomme de xanthane a été dispersée dans l'eau puis mélangée sous agitation avec un agitateur IKA (marque déposée) à une vitesse de 2500 tours/min jusqu'à obtenir une dispersion homogène.
- Les conservateurs et la glycérine ont été ensuite ajoutés au mélange obtenu précédemment,
- En parallèle, et si requis dans la composition, une phase « ascorbyl glucoside » a été préparée par pesée du glucoside d'ascorbyle puis addition à un mélange eau, soude, acide citrique et citrate de sodium. Cette phase a été ajoutée au mélange aqueux comprenant de la gomme de xanthane, les conservateurs et la glycérine
- L'extrait de fleurs de *Spiraea ulmaria* et l'extrait de racines de *Morus alba* ont été ensuite ajoutés au mélange, et
- le parfum après solubilisation dans du polysorbate 20 a été rajouté au mélange sous agitation avec un agitateur IKA (marque déposée) à une vitesse de 900 tr/min. Les concentrations ci-dessous sont exprimées en g pour 100 g de composition.

Les tableaux 1, 2 et 3 résument les ingrédients et quantités desdits ingrédients dans les compositions obtenues comprenant des actifs blanchissants (tableaux 1, 2 et 3).

**Tableau 1 : composition cosmétique éclaircissante, sérum, comprenant un extrait de Morus alba, de Spiraea ulmaria et de l'acide salicylique**

| **Ingrédients** | **Quantité en poids (grammes) pour 100 grammes de composition** |
|---|---|
| Xanthan gum | 0,3 |
| Hydroxide de sodium | 0,24 |
| Acide citrique | 0,02 |
| Ascorbyl Glucoside | 2 |
| Glycérine | 2 |
| éthyle hexyl glycérine | 0,3 |
| Extrait aqueux de fleurs de Spiraea ulmaria | 1 |
| Extrait aqueux de racines de Morus alba | 1 |
| Polysorbate 20 | 0,8 |
| Parfum | 0,1 |
| acide salicylique | 0,1 |
| Eau | QSP |

**Tableau 2 : crème cosmétique éclaircissante comprenant un extrait de Morus alba, de Spiraea ulmaria et de l'acide salicylique**

| **Ingrédients** | **Quantité en poids (grammes) pour 100 grammes de composition** |
|---|---|
| Dimethicone | 2 |
| Xanthan gum | 0,1 |
| Alcool cétylique | 0,5 |
| Palmitate de cétyle | 0,5 |
| Cyclopentasiloxane | 2 |
| Octyldodecanol | 2 |
| myristate octyldodecyle | 3 |
| Triglycéride caprylique/caprique | 3 |
| Stéarate d'éthyle hexyle | 2 |
| Chlorphénesine | 0,26 |
| Copolymère réticulé d'acrylate - alkyle en C₁₀₋₃₀ acrylate | 0,8 |
| Ascorbyl glucoside | 2 |
| Hydroxide de Sodium | 0,25 |
| Citrate de Sodium | 0,3 |
| Extrait aqueux de fleurs de Spirae ulmaria | 1 |
| Extrait aqueux de racines de Morus alba | 1 |
| Parfum | 0,08 |
| acide salicylique | 0,1 |
| Eau | QSP |

**Tableau 3: composition cosmétique éclaircissante, sérum, comprenant un extrait de Morus alba, de Spiraea ulmaria et de l'acide salicylique**

| **Ingrédients** | **Quantité en poids (grammes) pour 100 grammes de composition** |
|---|---|
| Xanthan gum | 0,3 |
| Glycérine | 2 |
| éthyle hexyl glycérine | 0,3 |
| Extrait aqueux de fleurs de Spiraea ulmaria | 1 |
| Extrait aqueux de racines de Morus alba | 1 |
| Polysorbate 20 | 0,8 |
| Parfum | 0,1 |
| acide salicylique | 0,1 |
| Eau | QSP |

### Exemple 2 : effet éclaircissant de compositions selon l'invention

Dans cet exemple, l'efficacité des actifs blanchissants a été évaluée au moyen de tests cellulaires, en particulier concernant l'effet sur la production de mélanine par des mélanocytes.

Dans cet exemple, l'extrait de racines de Mûrier blanc (Morus alba) était le *Souhakuhi Liquid BG* (nom INCI: *Morus alba* root extract (and) water (and) butylene glycol), l'extrait de fleurs de reine des prés (spiraea ulmaria) était le *Meadowsweet Liquid BG* (nom INCI: *Spiraea Ulmaria* Flower Extract (and) water (and) butylene glycol), l'acide kojique était l'acide kojique commercialisé par Sigma Aldrich sous la référence commerciale K-3125, la Vitamine C était l'Ascorbyl 2 glucoside commercialisé par la société DKSH sous la référence commerciale « Ascorbyl Acid 2 glucoside », l'acide salicylique était la molécule commercialisée par la société Cooper (réf 5 995 012, CAS 69-72-7)

Pour la réalisation des expériences, les cellules utilisées étaient des lignées cellulaires de souris, en particulier une lignée cellulaire de mélanocytes B16 de souris. Les conditions de culture des cellules était une température de 37°C sous une atmosphère de 5% en CO₂ dans un milieu de culture DMEM (sans rouge de phénol, comprenant 1g/l de glucose, supplémenté avec 2mM de L-glutamine, 50 U/ml de péniciline - 50µg/ml de Streptomycine, 10% v/v de sérum de veau foetal et 3g/l de glucose).

Le tableau 4 ci-dessous résume les composés et mélange de composés utilisés dans cet exemple ainsi que la concentration de chaque composé utilisé.

| Composés/mélange de composés | Concentrations testées en % en poids par rapport au poids total de la composition |
|---|---|
| Vitamine C | 0,125% ; 0,25% ; 0,5% |
| Acide salicylique | 0,0025%; 0,005%; 0,01 % |
| extrait de racines de Mûrier blanc | 0,25% ; 0,5%, 1% |
| extrait de fleurs de reine des prés | 0,25% ; 0,5%, 1% |
| Mélange extrait de racines de Mûrier blanc et extrait de fleurs de reine des prés | 0,25% et 0,25% ; |
| | 0,5% et 0,5% ; |
| | 1% et 1% |
| Mélange acide salicylique, extrait de racines de Mûrier blanc et extrait de fleurs de reine des prés | 0,0025%, 0,25% et 0,25% ; |
| | 0,005%, 0,5% et 0,5% ; |
| | 0,01%, 1% et 1% |
| Mélange Vitamine C, acide salicylique, extrait de racines de Mûrier blanc et extrait de fleurs de reine des prés | 0,125%, 0,0025%, 0,25% et 0,25%; |
| | 0,25%, 0,005%, 0,5% et 0,5%; |
| | 0,50%, 0,01 %, 1% et 1% |

Pour la réalisation des expériences, les mélanocytes ont été ensemencés dans des plaques 96 puits et cultivés pendant 24 heures. Le milieu de culture a été ensuite remplacé avec un milieu de culture comprenant un analogue stable de l'alpha-MSH, à savoir le sel de [Nle4, D-Phe7]-alpha-mélanocyte stimulating hormone (NDP-MSH) à une concentration de 10⁻⁷M et les composés/mélanges ou le composé témoin : (l'acide kojique à une concentration 3 mM) indépendamment introduits dans le milieu. Les cellules ont été ensuite incubées pendant 72 heures. En parallèle, du milieu sans cellules mais comprenant les composés/mélanges ou le composé témoin ont été évalués afin de déterminer, le cas échéant, toute interférence possible. L'ensemble des tests ont été réalisés en triplicate afin de confirmer les résultats obtenus.

La mesure de la quantité totale, c'est-à-dire intra et extracellulaire, de mélanine a été effectuée par mesure avec un spectrophotomètre de l'absorbance de la mélanine à une longueur d'onde de 405nm avec une courbe étalon et ceci pour chaque échantillon.

Les résultats obtenus sont représentés sur les figures 1 à 3 montrant le pourcentage d'inhibition de la mélanine en fonction de chaque composé et de sa concentration.

En particulier, la figure 1 démontre clairement l'efficacité dépigmentante de l'acide kojique, référence bien connue de l'homme de l'art. Ce résultat valide l'essai mis en oeuvre sur les mélanocytes pour évaluer l'efficacité des actifs blanchissants de la présente invention. Les résultats obtenus et représentés sur la figure 1 démontrent clairement que l'extrait de mûrier blanc ou l'extrait de reine des prés seul n'inhibe pas la production de mélanine de manière non significative. Au contraire, ces extraits seuls provoquent une légère stimulation de la production de mélanine de manière non significative.

Toutefois et de manière inattendue, la combinaison de l'extrait de reine des prés, de mûrier blanc et d'acide salicylique inhibe de manière significative la stimulation de la production de mélanine. En particulier, les résultats obtenus et décrits sur la figure 1 démontrent que le mélange comprenant un extrait de reine des prés, de mûrier blanc et d'acide salicylique a un effet synergique et inattendu concernant l'inhibition de la production de mélanine. Ces résultats et cette figure démontrent donc clairement qu'une composition cosmétique comprenant un extrait de reine des prés, de mûrier blanc et d'acide salicylique permet d'éclaircir la peau, de traiter les taches de vieillesse, les mélasmas, les lentigos et le chloasma.

En outre, les résultats obtenus représentés sur la figure 2 démontrent clairement qu'une composition comprenant un extrait de reine des prés, de mûrier blanc et d'acide salicylique lorsqu'elle est associée avec la vitamine C permet d'augmenter significativement l'inhibition de la production de mélanine. En particulier, la figure 2 démontre clairement que cette composition permet d'inhiber de plus de 100% la production de mélanine.

En d'autres termes, les résultats représentés sur la figure 2 démontrent donc clairement que la composition selon l'invention permet avantageusement, notamment de part l'effet synergique de la composition, d'assurer une efficacité blanchissante supérieure à celle apportée ou pas par les actifs de la composition pris isolément ; de diminuer les concentrations de principes actifs cosmétiques nécessaires pour obtenir un effet éclaircissant identique diminuant ainsi les risques de réactions secondaires et/ou de sensibilisations à ces principes actifs.

En outre, les résultats représentés sur la figure 3 démontrent également clairement que l'extrait de reine des prés, de mûrier blanc, l'acide salicylique et la vitamine C ont un effet synergique dans l'inhibition de la production de mélanine, en particulier pour les concentrations les plus faibles ou les plus fortes allant jusqu'à une inhibition de plus de 110 % de la production de la mélanine.

En d'autres termes, la figure 3 démontre donc clairement que la composition selon l'invention permet avantageusement, notamment de par l'effet synergique, de diminuer les concentrations de principes actifs cosmétiques nécessaires pour obtenir un effet éclaircissant diminuant ainsi les risques de réactions secondaires et/ou de sensibilisation à ces principes actifs.

Ainsi, cet exemple démontre clairement que :
- la composition selon l'invention permet avantageusement, notamment de part l'effet synergique, de diminuer les concentrations de principes actifs cosmétiques nécessaires pour obtenir un effet éclaircissant identique diminuant ainsi les risques de réactions secondaires et/ou de sensibilisations à ces principes actifs.
- l'effet synergique de l'extrait de reine des prés, de mûrier blanc, de l'acide salicylique et de la vitamine C permet également avantageusement de diminuer la fréquence d'application de la composition et/ou d'augmenter significativement, à des concentrations identiques, l'effet éclaircissant par rapport aux compositions de l'état de la technique
- La composition comprenant au moins un extrait de reine des prés, au moins un extrait de mûrier blanc et de l'acide salicylique est efficace dans un traitement cosmétique non thérapeutique notamment des taches de vieillesse, des mélasmas, des lentigos et du chloasma.
- la composition permet également de raviver l'éclat de la peau notamment en estompant les taches pigmentaires et avantageusement en diminuant le ou les différence(s) de couleur entre les taches pigmentaires et le reste de la carnation.

## Revendications

1. Utilisation cosmétique d'une composition cosmétique comprenant au moins un extrait de reine des prés, au moins un extrait de mûrier blanc et de l'acide salicylique pour éclaircir la peau.

2. Utilisation de la composition selon la revendication 1, dans laquelle un extrait de reine des prés est un extrait choisi parmi un extrait aqueux, un extrait glycolique un extrait huileux, une huile essentielle ou un mélange de ceux-ci.

3. Utilisation de la composition selon la revendication 1 ou 2, dans laquelle un extrait de mûrier blanc est un extrait choisi parmi un extrait aqueux, un extrait huileux, un extrait glycolique, ou un mélange de ceux-ci.

4. Utilisation de la composition selon l'une quelconque des revendications 1 à 3, dans laquelle l'extrait de reine des prés est présent dans la composition cosmétique à une concentration de 0,01 à 10 % en poids par rapport au poids total de ladite composition.

5. Utilisation de la composition selon l'une quelconque des revendications 1 à 4, dans laquelle l'extrait de mûrier blanc est présent dans la composition cosmétique à une concentration de 0,01 à 10 % en poids par rapport au poids total de ladite composition.

6. Utilisation de la composition selon l'une quelconque des revendications 1 à 5, dans laquelle l'acide salicylique est présent dans la composition cosmétique à une concentration de 0,001 à 2 % en poids par rapport au poids total de ladite composition.

7. Utilisation de la composition selon l'une quelconque des revendications 1 à 6, dans laquelle la composition comprend en outre de la Vitamine C ou ses dérivés.

8. Utilisation de la composition selon l'une quelconque des revendications 1 à 7, dans laquelle la vitamine C est présente dans la composition cosmétique à une concentration de 0,01 à 10 % en poids par rapport au poids total de ladite composition.

9. Utilisation de la composition selon l'une quelconque des revendications précédentes, ladite composition étant sous une forme choisie parmi une crème, un gel, une lotion, un onguent, une huile, un lait, un masque, un sérum, un patch.

10. Utilisation cosmétique selon l'une quelconque des revendications précédentes, pour traiter les taches de vieillesse, les mélasmas, les lentigos et/ou le masque de grossesse ou chloasma.

## Patentansprüche

1. Kosmetische Verwendung einer kosmetischen Zusammensetzung, die mindestens einen Extrakt von Echtem Mädesüß, mindestens einen Extrakt von Weißer Maulbeere und Salicylsäure umfasst, zum Aufhellen der Haut.

2. Verwendung der Zusammensetzung nach Anspruch 1, wobei ein Extrakt von Echtem Mädesüß ein Extrakt ist, der aus einem wässrigen Extrakt, einem glykolischen Extrakt, einem Ölextrakt, einem ätherischen Öl oder einem Gemisch von diesen ausgewählt ist.

3. Verwendung der Zusammensetzung nach Anspruch 1 oder 2, wobei ein Extrakt von Weißer Maulbeere ein Extrakt ist, der aus einem wässrigen Extrakt, einem Ölextrakt, einem glykolischen Extrakt oder einem Gemisch von diesen ausgewählt ist.

4. Verwendung der Zusammensetzung nach einem der Ansprüche 1 bis 3, wobei der Extrakt von Echtem Mädesüß in der kosmetischen Zusammensetzung in einer Konzentration von 0,01 bis 10 Gew.-%, bezogen auf das Gesamtgewicht der Zusammensetzung, vorliegt.

5. Verwendung der Zusammensetzung nach einem der Ansprüche 1 bis 4, wobei der Extrakt von Weißer Maulbeere in der kosmetischen Zusammensetzung in einer Konzentration von 0,01 bis 10 Gew.-%, bezogen auf das Gesamtgewicht der Zusammensetzung, vorliegt.

6. Verwendung der Zusammensetzung nach einem der Ansprüche 1 bis 5, wobei die Salicylsäure in der kosmetischen Zusammensetzung in einer Konzentration von 0,001 bis 2 Gew.-%, bezogen auf das Gesamtgewicht der Zusammensetzung, vorliegt.

7. Verwendung der Zusammensetzung nach einem der Ansprüche 1 bis 6, wobei die Zusammensetzung außerdem Vitamin C oder dessen Derivate umfasst.

8. Verwendung der Zusammensetzung nach einem der Ansprüche 1 bis 7, wobei das Vitamin C in der kosmetischen Zusammensetzung in einer Konzentration von 0,01 bis 10 Gew.-%, bezogen auf das Gesamtgewicht der Zusammensetzung, vorliegt.

9. Verwendung der Zusammensetzung nach einem der vorhergehenden Ansprüche, wobei die Zusammensetzung in einer aus einer Creme, einem Gel, einer Lotion, einer Salbe, einem Öl, einer Milch, einer Maske, einem Serum, einem Pflaster ausgewählten Form vorliegt.

10. Kosmetische Verwendung nach einem der vorhergehenden Ansprüche zur Behandlung von Altersflecken, Melasmen, Lentigos und/oder einer Schwangerschaftsmaske oder von Chloasma.

## Claims

1. Cosmetic use of a cosmetic composition comprising at least one meadowsweet extract, at least one white mulberry extract and salicylic acid to lighten the skin.

2. Use of the composition according to claim 1, wherein a meadowsweet extract is an extract selected among an aqueous extract, a glycolic extract, an oily extract, an essential oil or a mixture thereof.

3. Use of the composition according to claim 1 or 2, wherein a white mulberry extract is an extract selected among an aqueous extract, an oily extract, a glycolic extract or a mixture thereof.

4. Use of the composition according to any one of claims 1 to 3, wherein the meadowsweet extract is present in the cosmetic composition at a concentration from 0.01 to 10% by weight relative to the total weight of said composition.

5. Use of the composition according to any one of claims 1 to 4, wherein the white mulberry extract is present in the cosmetic composition at a concentration from 0.01 to 10% by weight relative to the total weight of said composition.

6. Use of the composition according to any one of claims 1 to 5, wherein the salicylic acid is present in the cosmetic composition at a concentration from 0.001 to 2% by weight relative to the total weight of said composition.

7. Use of the composition according to any one of claims 1 to 6, wherein the composition also comprises vitamin C or derivatives thereof.

8. Use of the composition according to any one of claims 1 to 7, wherein the vitamin C is present in the cosmetic composition at a concentration from 0.01 to 10% by weight relative to the total weight of said composition.

9. Use of the composition according to any one of the preceding claims, said composition being in a form selected among a cream, a gel, a lotion, a salve, an oil, a milk, a mask, a serum or a patch.

10. Use of the composition according to any one of the preceding claims, to treat age spots, melasmas, lentigines, and/or the mask of pregnancy or chloasma.
